# EUROPÄISCHE PATENTSCHRIFT

(11) **EP 1 782 856 B1**
(45) Veröffentlichungstag und Bekanntmachung des Hinweises auf die Patenterteilung: **06.01.2010**
(21) Anmeldenummer: 05023994.6
(22) Anmeldetag: 03.11.2005
(51) Int. Cl.: A61M 35/00, A61B 17/00, A61J 1/06, A61J 1/00, A61M 5/28, A61M 5/24

(54) **Appliziervorrichtung für eine fliessfähige Substanz**
Dispenser for a liquid product
Applicateur pour un produit liquide

(43) Veröffentlichungstag der Anmeldung: 09.05.2007
(73) Patentinhaber: Dentaco Dentalindustrie und -marketing GmbH, 61352 Bad Homburg (DE)
(72) Erfinder: Sògaro, Alberto C., 61476 Kronberg (DE)
(74) Vertreter: advotec.

(56) Entgegenhaltungen:
- DE-U1- 20 019 091
- US-A- 4 657 534
- US-A- 4 741 737
- US-A- 4 941 876

## Beschreibung

Die Erfindung betrifft eine Appliziervorrichtung für eine fließfähige Substanz, umfassend einen Trägerkörper, der mit einer Applikationseinrichtung versehen ist und der an der der Applikationseinrichtung abgewandten Seite einen zylindrischen Zapfen aufweist, der von einem Querkanal durchgriffen ist, von dem ein Axialkanal abzweigt, der zu der Applikationseinrichtung führt, wobei auf dem zylindrischen Zapfen eine zur Aufnahme der fließfähigen Substanz dienende Vorratseinrichtung angeordnet ist, die mindestens eine an der Innenseite angeordnete ringförmige Dichtlippe aufweist, die dichtend mit dem zylindrischen Zapfen des Trägerkörpers zusammenwirkt, wobei eine Aktivierung der Appliziervorrichtung durch ein Verschieben der Vorratseinrichtung auf dem zylindrischen Zapfen in Richtung der Applikationseinrichtung erfolgt.

Eine derartige Appliziervorrichtung ist aus der DE 200 19 091 U1 bekannt und dient insbesondere zum Applizieren pharmazeutischer oder kosmetischer Substanzen am menschlichen Körper. Die bekannte Appliziervorrichtung ist nach Art einer Minispritze ausgebildet und weist als Applikationseinrichtung eine kanülenförmige Spitze auf, über die die fließfähige Substanz ausgetragen werden kann. Zur Aktivierung wird die Vorratseinrichtung, die topfartig bzw. nach Art eines einseitig geschlossenen Röhrchens ausgebildet ist, auf dem zylindrischen Zapfen manuell so verschoben, dass der Zapfen als Kolben wirkt, der die in der Vorratseinrichtung vorgehaltene fließfähige Substanz verdrängt und über den Querkanal und den Axialkanal des Trägerkörpers zu der Applikationsspitze fördert. Bei der Aktivierung taucht die Seitenwand der Vorratseinrichtung in eine ringförmige Ausnehmung ein, die den zylindrischen Zapfen umgibt.

Die aus der DE 200 19 091 U1 bekannte Appliziervorrichtung hat den Nachteil, dass ein Feindosieren der fließfähigen Substanz nicht ohne Weiteres möglich ist, da sich ein kontrolliertes Verschieben der röhrchenförmigen Vorratseinrichtung auf dem zylindrischen Zapfen als schwierig erweist.

Der Erfindung liegt die Aufgabe zugrunde, eine gemäß der einleitend genannten Gattung ausgebildete Appliziervorrichtung zu schaffen, die sich gegenüber den Stand der Technik durch verbesserte Dosiereigenschaften auszeichnet.

Diese Aufgabe ist erfindungsgemäß durch die Appliziervorrichtung mit den Merkmalen des Patentanspruches 1 gelöst.

Die Erfindung besteht mithin darin, dass die Vorratseinrichtung einen zumindest bereichsweise elastisch verformbaren Aufnahmeabschnitt aufweist, der in Aktivierungsstellung der Appliziervorrichtung, in der die Dichtlippe an der dem Vorratsabschnitt abgewandten Seite der Öffnungen des Querkanals des zylindrischen Zapfens angeordnet ist und mithin eine Fließverbindung zwischen dem Aufnahmeabschnitt und dem Querkanal besteht, so manuell komprimiert werden kann, dass die fließfähige Substanz über die Appliziervorrichtung ausgetragen wird.

Das Prinzip der Appliziervorrichtung nach der Erfindung besteht mithin darin, dass nach der Aktivierung, d.h. nach dem Verschieben der Vorratseinrichtung auf dem zylindrischen Zapfen, das Ausbringen der fließfähigen Substanz über die Applikationseinrichtung durch ein seitliches manuelles Drücken des Aufnahmeabschnitts erfolgt. Ein weiterer Versatz der Applikationseinrichtung auf dem zylindrischen Zapfen ist hierzu nicht erforderlich. Der Zapfen wirkt also nicht als Verdrängungskolben für die fließfähige Substanz.

Die Appliziervorrichtung nach der Erfindung eignet sich insbesondere zum Applizieren pharmazeutischer oder kosmetischer Substanzen am menschlichen oder tierischen Körper und kann hierzu mit einer dem jeweiligen Anwendungsfall abgepassten Applikationseinrichtung versehen sein. Beispielsweise kann die Applikationseinrichtung eine Pipitierspitze darstellen oder auch einen Pinsel oder ein Schwämmchen umfassen. Eine pharmazeutische Substanz, die mittels der Vorrichtung nach der Erfindung applizierbar ist, ist beispielsweise ein Gewebekleber, ein Dentalkleber oder dergleichen.

Die Appliziervorrichtung nach der Erfindung ist insbesondere als Einwegvorrichtung ausgelegt, bei der die Vorratseinrichtung vorab befüllt ist. Im deaktivierten, eine Sperrstellung darstellenden Zustand ist die fließfähige Substanz durch die Dichtlippe gesichert in der Vorratseinrichtung gehalten. Dieser Zustand stellt in der Regel den Lieferzustand der Appliziervorrichtung dar. Der Anwender muss zur Aktivierung der Appliziervorrichtung nur ein teleskopartiges Zusammenschieben des Trägerkörpers und der Vorratseinrichtung der Appliziervorrichtung vornehmen, so dass ein Übergang von der geschlossenen Speicherposition in die offene Freigabeposition der Applikationseinrichtung bezüglich des Trägerkörpers erfolgt. Beim Aktivieren überstreicht die Dichtlippe die Öffnungen des Querkanals in dem Zapfen.

Der Aufnahmeabschnitt der Vorratseinrichtung kann in vielfältiger Weise ausgebildet sein. Beispielsweise hat der Aufnahmeabschnitt eine blasenartige, eine röhrchenartige, eine tubenartige, eine kegelartige oder auch eine wulstartige Form.

Die Vorratseinrichtung der Appliziervorrichtung nach der Erfindung kann eine oder auch mehrere Aufnahmekörper umfassen. In letzterem Fall kann in jedem der Körper eine Komponente eines Mehrkomponentensystems vorgehalten werden. Bei einer Aktivierung der Appliziervorrichtung ist es erforderlich, eine Fließverbindung zwischen den Aufnahmekörpern der Vorratseinrichtung herzustellen, so dass das vermischte Mehrkomponentensystem von einem Raum der Vorratseinrichtung aufgenommen wird, der von einer elastisch verformbaren Wandung des Aufnahmeabschnitts umgeben ist, welchen den Aufnahmeabschnitt zum Applizieren der fließfähigen Substanz kennzeichnet. Das gemischte Mehrkomponentensystem kann dann nach Komprimieren des Aufnahmeabschnitts zu dem Querkanal und von dort über den Axialkanal zu der Applikationseinrichtung gefördert werden.

Bei einer speziellen Ausführungsform der Appliziervorrichtung nach der Erfindung sind die Aufnahmekörper einer mehrere Aufnahmekörper aufweisenden Vorratseinrichtung zur Herstellung einer Fließverbindung teleskopierbar. Hierbei hat vorzugsweise einer der Aufnahmekörper einen zylindrischen Abschnitt, der innenseitig eine Dichtlippe hat, die mit einer zylindrischen Umfangsfläche des anderen Aufnahmekörpers zusammenwirkt, an dem Queröffnungen ausgebildet sind. Wenn die Dichtlippe die Queröffnungen überfährt, ist eine Fließverbindung zwischen den Aufnahmekörpern hergestellt.

Des Weiteren ist es denkbar, dass die Appliziervorrichtung zwei nebeneinander angeordnete Vorratseinrichtungen umfasst, die jeweils auf einem zylindrischen Zapfen angeordnet sind, der entsprechend der oben beschriebenen Art ausgebildet ist. Der Trägerkörper hat dann zwei im Wesentlichen parallele Axialkanäle, stromab derer ein statischer Mischer angeordnet sein kann.

Weitere Vorteile und vorteilhafte Ausgestaltungen des Gegenstandes nach der Erfindung sind der Beschreibung, der Zeichnung und den Patentansprüchen entnehmbar.

Sechs Ausführungsbeispiele einer Appliziervorrichtung nach der Erfindung sind in der Zeichnung schematisch vereinfacht dargestellt und werden in der nachfolgenden Beschreibung näher erläutert. Es zeigt:
- Fig. 1: einen Längsschnitt durch eine erste Ausführungsform einer Appliziervorrichtung für ein Einkomponentensystem in Deaktivierungsstellung;
- Fig. 2: die Appliziervorrichtung nach Fig. 1 in Aktivierungsstellung;
- Fig. 3: einen Längsschnitt durch eine zweite Ausführungsform einer Appliziervorrichtung für ein Einkomponentensystem in Deaktivierungsstellung;
- Fig. 4: die Appliziervorrichtung nach Fig. 3 in Aktivierungsstellung;

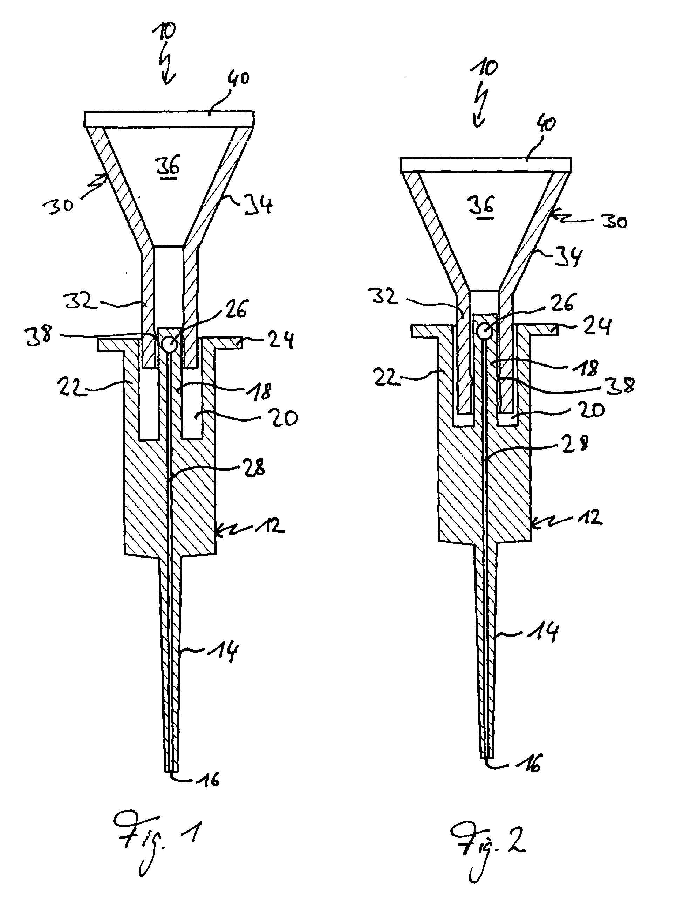

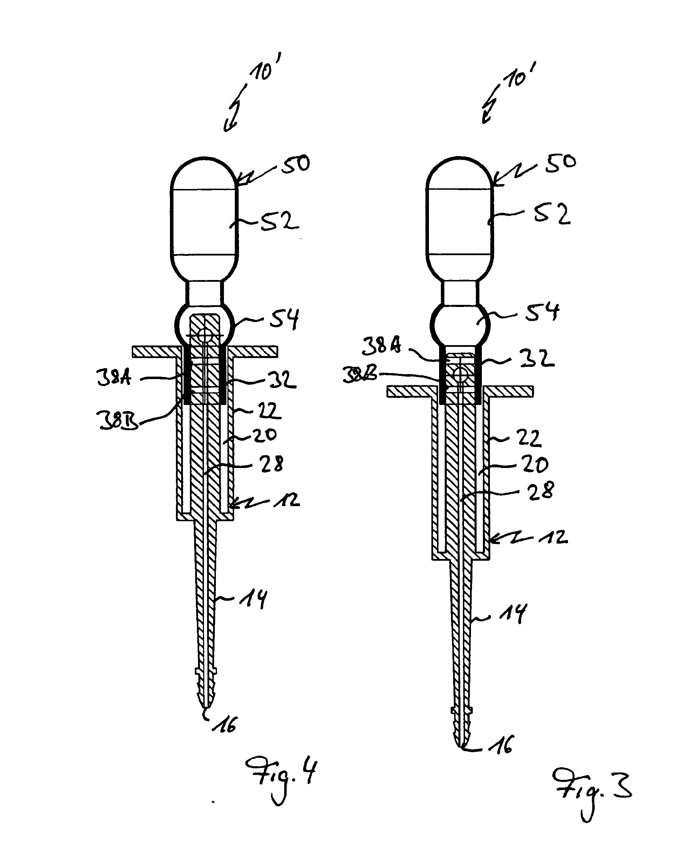

- Fig. 4: eine alternative Ausführungsform einer Vorratseinrichtung einer Appliziervorrichtung der in Fig. 1 dargestellten Art;

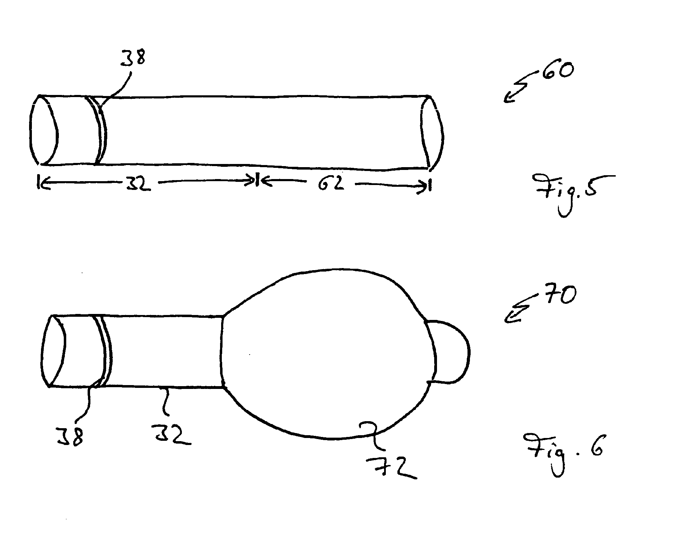

- Fig. 5: eine weitere Ausführungsform einer Vorratseinrichtung;
- Fig. 6: eine vierte Ausführungsform einer Vorratseinrichtung;

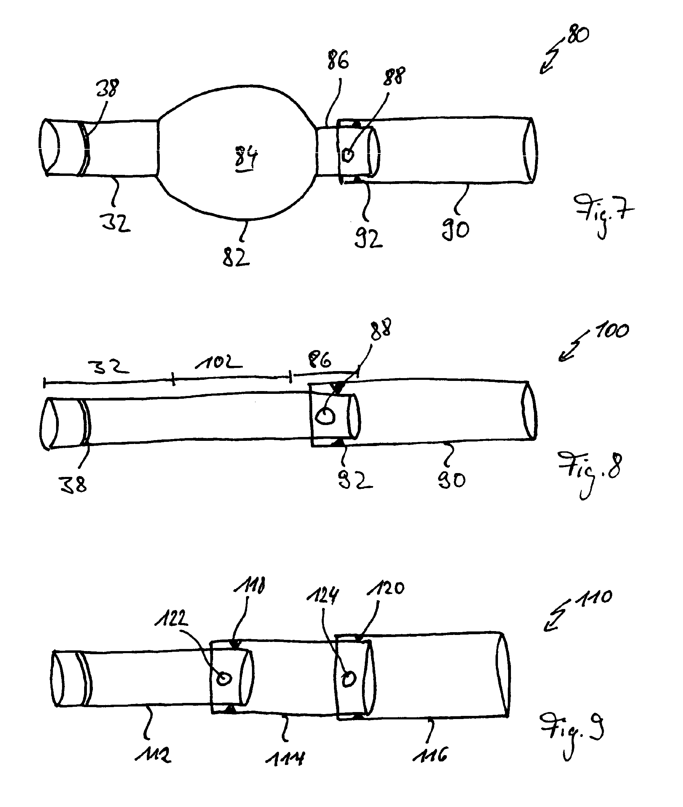

- Fig. 7: eine Vorratseinrichtung mit mehreren Aufnahmekammern;
- Fig. 8: eine alternative Ausführungsform einer Vorratseinrichtung mit mehreren Aufnahmekammern; und
- Fig. 9: eine letzte Ausführungsform einer Vorratseinrichtung mit mehreren Aufnahmekammern.

In den Figuren 1 und 2 ist eine Appliziervorrichtung 10 dargestellt, die beispielsweise zum Pipetieren einer fließfähigen Substanz dient und die einen Trägerkörper 12 umfasst, an dem eine als Spitze ausgebildete Appliziervorrichtung 14 einstückig angeformt ist. Die Appliziervorrichtung 14 hat an ihrer freien Stirnseite eine Austragöffnung 16 für die fließfähige Substanz.

An der der Appliziervorrichtung 14 abgewandten Seite weist der Trägerkörper 12 einen zylindrischen, kolbenartigen Zapfen 18 auf, der von einer ringförmigen Ausnehmung 20 umgeben ist, welche außen wiederum von einer Umfangswand 22 begrenzt ist. An der Umfangswand 22 ist wiederum ein als Greifhilfe dienender Kragen 24 angeformt.

In dem der Applikationseinrichtung 14 abgewandten Endbereich ist der Zapfen 18 von einem sich in radialer Richtung erstreckenden Querkanal 26 durchgriffen. Von dem Querkanal 26 zweigt wiederum ein in der Achse des Zapfens 18 liegender Axialkanal 28 ab, der sich bis zu der Austragsöffnung 16 der Applikationseinrichtung 14 erstreckt.

Des Weiteren umfasst die Appliziervorrichtung 10 eine Vorratseinrichtung 30, in der die fließfähige Substanz vorgehalten werden kann. Die Vorratseinrichtung 30 umfasst einen rohrförmigen Führungsabschnitt 32 und einen sich an der der Applikationseinrichtung 14 abgewandten Seite anschließenden Aufnahmeabschnitt 34, in dem eine Aufnahmekammer 36 für die fließfähige Substanz ausgebildet ist.

Der Führungsabschnitt 32 weist an seiner Innenseite eine ringförmige Dichtlippe 38 auf, die gleitend an der Umfangsfläche des zylindrischen Zapfens 18 angeordnet ist.

Der Aufnahmeabschnitt 36 ist tubenartig ausgebildet und weist in seinem dem Zapfen 18 abgewandten Endbereich eine Siegelnaht 40 auf.

Die Vorratseinrichtung 30 ist aus einem elastisch verformbaren Werkstoff gefertigt, so dass der Aufnahmeabschnitt 34 manuell zusammengedrückt werden kann.

In der in Fig. 1 dargestellten Stellung der Vorratseinrichtung 30 ist die Dichtlippe 38 in einem Endbereich der Umfangsfläche des Zapfens 18 angeordnet, so dass ein Fluidstrom zwischen der Aufnahmekammer 36 und dem Querkanal 26 gesperrt ist. Zur Aktivierung der Appliziervorrichtung 10 wird die Vorratseinrichtung 30 in ihre offene Freigabestellung verfahren, die in Fig. 2 dargestellt ist. Hierbei überfährt die Dichtlippe 38 die Querbohrung 26, so dass über einen Ringspalt zwischen dem zylindrischen Zapfen 18 und dem Führungsabschnitt 32 der Vorratseinrichtung 30 eine Fließverbindung zwischen der Aufnahmekammer 36 und dem Querkanal 26 hergestellt wird. Durch einen seitlichen manuellen Druck, der auf den Aufnahmeabschnitt 34 ausgeübt wird, kann dann die in der Aufnahmekammer 36 vorgehaltene fließfähige Substanz aus der Aufnahmekammer 36 verdrängt und über den Querkanal 26 und den Axialkanal 28 zu der Austragöffnung 16 der Applikationseinrichtung 14 gefördert und appliziert werden.

In den Figuren 3 und 4 ist eine alternative Ausführungsform einer Appliziervorrichtung 10' dargestellt, die eine Vorratseinrichtung 50 umfasst, die anstelle der in Fig. 1 dargestellten Vorratseinrichtung auf einen Trägerkörper 12 der in Fig. 1 dargestellten Art aufgesetzt werden kann. Die Vorratseinrichtung 50 umfasst einen rohrförmigen Führungsabschnitt 32, der innenseitig zwei ringförmige Dichtlippen 38A und 38B aufweisen, die mit dem zylindrischen Zapfen 18 des Trägerkörpers 12 zusammenwirken. An dem den Dichtlippen 38A und 38B abgewandten Ende schließt sich an den Führungsabschnitt 32 ein blasenartiger Aufnahmeabschnitt 52 an, in dem eine zu applizierende, fließfähige Substanz vorgehalten wird und der elastisch komprimierbar ausgebildet ist.

In einem mittleren Bereich des Führungsabschnitts 32 ist des Weiteren ein aufgeweiteter Bereich 54 ausgebildet, der in Aktivierungsstellung der Vorratseinrichtung 50 auf dem zylindrischen Zapfen des Trägerkörpers 12 in Höhe von dessen Querkanal 26 angeordnet ist und das Strömungsverhalten der fließfähigen Substanz verbessert und als Anschlag beim Aktivieren der Appliziervorrichtung 10' dient.

Die Funktion der Vorratseinrichtung 50 und deren Zusammenwirken mit dem Trägerkörper 18 sind entsprechend zu der Ausführungsform nach den Figuren 1 und 2.

In Fig. 5 ist eine weitere Ausführungsform einer Vorratseinrichtung 60 dargestellt, die in Verbindung mit einem Trägerkörper 12 der in Fig. 1 dargestellten Art eingesetzt werden kann.

Die Vorratseinrichtung 60 ist aus einem elastisch verformbaren Röhrchen gebildet, das ein offenes und ein geschlossenes Ende aufweist. Im Bereich des offenen Endes ist an der Innenseite des Röhrchens eine Dichtlippe 38 angeordnet, die entsprechend den Ausführungsformen nach den Figuren 1 bis 3 mit dem zylindrischen Zapfen des Trägerkörpers zusammenwirkt und einem Führungsabschnitt 32 der Vorratseinrichtung 60 zugeordnet ist. Die Vorratseinrichtung 60 ist länger ausgebildet als der zylindrischen Zapfen 18 des zugeordneten Trägerkörpers 12 der Appliziervorrichtung, so dass auch im aktivierten Zustand der betreffenden Appliziervorrichtung anschließend an den Führungsabschnitt 32 ein Aufnahmeabschnitt 62 verbleibt, in dem die fließfähige Substanz nach Aktivierung der betreffenden Appliziervorrichtung angeordnet ist. Die fließfähige Substanz wird damit nicht durch eine Kolbenwirkung des Zapfens 18 des Trägerkörpers 12 aus der Vorratseinrichtung 60 ausgetrieben. Der Aufnahmeabschnitt 62 kann vielmehr durch seitlichen manuellen Druck komprimiert werden, wodurch die fließfähige Substanz über den Querkanal 26 und den Axialkanal 28 des Trägerkörpers 12 zu der Applikationseinrichtung 14 gefördert und über diese appliziert wird.

In Fig. 6 ist eine weitere Ausführungsform einer Vorratseinrichtung 70 dargestellt, die sich von derjenigen nach den Figuren 1 und 2 lediglich dadurch unterscheidet, dass sie statt einen tubenförmigen einen blasenförmigen Aufnahmeabschnitt 72 aufweist.

In Fig. 7 ist eine Vorratseinrichtung 80 dargestellt, die ebenfalls zur Anwendung in Verbindung mit einem Trägerkörper 12 der in Fig. 1 dargestellten Art ausgelegt ist. Die Vorratseinrichtung 80 ist für ein Zweikomponentensystem ausgelegt, dessen einzelnen Komponenten erst unmittelbar vor der Applikation vermengt werden. Hierzu umfasst die Vorratseinrichtung 80 einen ersten Aufnahmekörper 82 mit einem Führungsabschnitt 32 zum Zusammenwirken mit dem zylindrischen Zapfen 18 des Trägerkörpers 12 in der in Verbindung mit Fig. 1 beschriebenen Weise. Der Aufnahmekörper 82 weist des Weiteren einen Aufnahmeabschnitt 84 auf, in dem eine Komponenten des Zweikomponentensystems vor dem Vermischen vorgehalten wird. Der Aufnahmeabschnitt 84 ist elastisch komprimierbar ausgebildet. An der dem Führungsabschnitt 32 abgewandten Seite umfasst der Aufnahmekörper 82 einen weiteren Führungsabschnitt 86, der an seiner Umfangsfläche mit Queröffnungen 88 versehen und stirnseitig verschlossen ist. Auf dem Führungsabschnitt 86 sitzt ein topfartiger, zweiter Aufnahmekörper 90, der mit seinem dem Boden abgewandten Endbereich auf dem Führungsabschnitt 86 verschiebbar geführt ist und in diesem Bereich eine innere Dichtlippe 92 aufweist.

Zur Aktivierung wird der zweite Aufnahmekörper 90 gegenüber dem ersten Aufnahmekörper 82 teleskopiert, so dass über einen Ringspalt zwischen dem Führungsabschnitt 86 des ersten Aufnahmekörpers 82 und dem zweiten Aufnahmekörper 90 sowie die Queröffnungen 88 eine Fließverbindung zwischen dem Innenraum des zweiten Aufnahmekörpers 90 und dem Innenraum des ersten Aufnahmekörpers 82 hergestellt ist. Die Substanz in dem zweiten Aufnahmeabschnitt 90 kann so in den ersten Aufnahmekörper 82 strömen und sich dort mit der anderen Komponente des Zweikomponentensystems vermengen.

Durch entsprechendes Verschieben der Vorratseinrichtung 80 auf dem zylindrischen Zapfen 18 des Trägerkörpers 12 wird dann auch eine Fluidverbindung zwischen dem Querkanal 26 des Trägerkörpers 12 und dem Aufnahmeraum des Aufnahmeabschnitts 84 hergestellt. Anschließend kann durch manuelles Zusammendrücken des Aufnahmeabschnitts 84 das Zweikomponentensystem 14 über die Applikationseinrichtung 14 appliziert werden.

Die in Fig. 8 dargestellte Ausführungsform einer Vorratseinrichtung 100 entspricht im Wesentlichen derjenigen der Vorratseinrichtung nach Fig. 6 und unterscheidet sich von dieser lediglich dadurch, dass der erste Aufnahmekörper 82 nach Art eines einseitig verschlossenen Röhrchens ausgebildet ist, das sowohl den Führungsabschnitt 32 als auch einen sich an diesen anschließenden, seitlich komprimierbaren Aufnahmeabschnitt 102 umfasst.

In Fig. 9 ist eine Vorratseinrichtung 110 dargestellt, die ebenfalls zur getrennten Bevorratung zweier Komponenten eines Zweikomponentensystems ausgebildet ist.

Die Vorratseinrichtung 110 weist drei Teilstücke 112, 114 und 116 auf, die jeweils röhrchenartig ausgebildet sind und ein offenes und ein geschlossenes Ende aufweisen. Im Bereich des offenen Endes ist an der Innenseite der Röhrchen 112, 114 und 116 jeweils eine Dichtlippe 38, 118 bzw. 120 ausgebildet, die mit der Umfangsfläche des Trägerkörpers 12 bzw. des benachbarten Teilstücks 112 bzw. 114 zur Freigabe des Querkanals 26 des Zapfens 18 des Trägerkörpers 12 bzw. von Queröffnungen 122 bzw. 124 des benachbarten Teilstücks 112 bzw. 114 zusammen wirkt.

Zumindest das Teilstück 112 ist in Querrichtung komprimierbar ausgebildet, so dass das in diesem Teilstück 112 enthaltene Komponentengemisch des Zweikomponentensystems durch manuellen Druck auf dieses Teilstück 112 aus der Vorratseinrichtung 110 verdrängt und über den Trägerkörper 12 und die Applikationseinrichtung 14 appliziert werden kann.

## Patentansprüche

1. Appliziervorrichtung für eine fließfähige Substanz, umfassend einen Trägerkörper (12), der mit einer Applikationseinrichtung (14) versehen ist und der an der der Applikationseinrichtung (14) abgewandten Seite eine zylindrischen Zapfen (18) aufweist, der von einem Querkanal (26) durchgriffen ist, von dem ein Axialkanal (28) abzweigt, der zu der Applikationseinrichtung (14) führt, wobei auf dem zylindrischen Zapfen (18) eine Vorratseinrichtung (30, 50, 60, 70, 80, 90, 100, 110) verschiebbar angeordnet ist, die an ihrer Innenseite eine ringförmige Dichtlippe (38) aufweist, die dichtend mit dem zylindrischen Zapfen (18) zusammenwirkt, wobei eine Aktivierung der Appliziervorrichtung durch ein Verschieben der Vorratseinrichtung (30, 50, 60, 70, 80, 100, 110) auf dem zylindrischen Zapfen (18) in Richtung der Applikationseinrichtung (14) zur Freigabe einer Fließverbindung zwischen der Vorratseinrichtung (30, 50, 60, 70, 80, 100, 110) und dem Querkanal (26) erfolgt, **dadurch gekennzeichnet, dass** die Vorratseinrichtung (30, 50, 60, 70, 80, 100, 110) einen zumindest bereichsweise elastisch verformbaren Aufnahmeabschnitt (34, 52, 62, 72, 82, 112) aufweist, so dass, wenn in Aktivierungsstellung der Appliziereinrichtung eine Fließverbindung zwischen dem Querkanal (26) und dem Aufnahmeabschnitt (34, 52, 62, 72, 82, 112) besteht, durch manuelles Komprimieren des elastisch verformbaren Bereichs des Aufnahmeabschnitts (34, 52, 62, 72, 82, 112) die fließfähige Substanz über die Applikationseinrichtung (14) ausgetragen wird.

2. Appliziervorrichtung nach Anspruch 1, **dadurch gekennzeichnet, dass** der elastisch verformbare Bereich des Aufnahmeabschnitts (52, 72, 82) blasenartig ausgebildet ist.

3. Appliziervorrichtung nach Anspruch 1, **dadurch gekennzeichnet, dass** der elastisch verformbare Bereich des Aufnahmeabschnitts röhrchenartig ausgebildet ist.

4. Appliziervorrichtung nach Anspruch 1, **dadurch gekennzeichnet, dass** der elastisch verformbare Bereich des Aufnahmeabschnitts tubenartig ausgebildet ist.

5. Appliziervorrichtung nach einem der Ansprüche 1 bis 4, **dadurch gekennzeichnet, dass** der Führungsabschnitt (32) einen aufgeweiteten Bereich (54) hat, der bei Aktivierung der Appliziervorrichtung in Höhe des Querkanals (26) des Zapfens (18) liegt.

6. Appliziervorrichtung nach einem der Ansprüche 1 bis 5, **dadurch gekennzeichnet, dass** die Vorratseinrichtung (80, 100, 110) mindestens zwei Aufnahmekörper für jeweils eine Komponente eines Mehrkomponentensystems umfasst.

7. Appliziervorrichtung nach Anspruch 6, **dadurch gekennzeichnet, dass** die mindestens zwei Aufnahmekörper zur Herstellung einer Fließverbindung teleskopierbar sind, wobei zumindest einer der Aufnahmekörper innenseitig eine Dichtlippe (88, 118, 120) hat, die mit einer Umfangsfläche des anderen Aufnahmekörpers zusammenwirkt.

8. Appliziervorrichtung nach einem der Ansprüche 1 bis 7, **dadurch gekennzeichnet, dass** der zylindrischen Zapfen (18) mindestens eine Ringnut aufweist, die mit der Dichtlippe (38) zusammenwirkt und eine Deaktivierungsstellung und/oder eine Aktivierungsstellung der Aufnahmeeinrichtung definiert.

## Claims

1. An applicator device for a free-flowing substance comprising a carrier body (12) which is provided with an applicator (14) and which, at the distal end of the applicator (14) has a cylindrical pin (18) which is penetrated by a transverse channel (26), from which an axial channel (28) branches off, which leads to the applicator (14), whereby on the cylindrical pin (18) a reservoir device (30, 50, 60, 70, 80, 90, 100, 110) can be displaced which on its inside has a ring-shaped edge seal (38) which sealingly interacts with the cylindrical pin (18), whereby the applicator device is activated by the displacement of the reservoir device (30, 50, 60, 70, 80, 100, 110) on the cylindrical pin (18) toward the applicator (14) to open a flow connection between the reservoir device (30, 50, 60, 70, 80, 100, 110) and the transverse channel (26), **characterized in that** the reservoir device (30, 50, 60, 70, 80, 100, 110) has a receptacle segment (34, 52, 62, 72, 82, 112) that is elastically deformable at least in portions so that, when a flow connection between the transverse channel (26) and the receptacle segment (34, 52, 62, 72, 82, 112) exists in the activation position of the applicator device, the free-flowing substance is discharged via the applicator (14) by manual compression of the elastically deformable portion of the receptacle segment (34, 52, 62, 72, 82, 112).

2. The applicator device as recited in claim 1, **characterized in that** the elastically deformable portion of the receptacle segment (52, 72, 82) is realized in the form of a bubble.

3. The applicator device as recited in claim 1, **characterized in that** the elastically deformable portion of the receptacle segment is realized in the form of a small tube.

4. The applicator device as recited in claim 1, **characterized in that** the elastically deformable portion of the receptacle segment is realized in the form of a collapsible tube.

5. The applicator device as recited in any of the claims 1 to 4, **characterized in that** the guide segment (32) has a widened portion (54) which during the activation of the applicator device lies at the level of the transverse channel (26) of the pin (18).

6. The applicator device as recited in any of the claims 1 to 5, **characterized in that** the reservoir device (80, 100, 10) comprises at least two receptacle bodies, one for each component of a multiple-component system.

7. The applicator device as recited in claim 6, **characterized in that** the at least two receptacle bodies can be telescoped for the creation of a flow connection, whereby at least one of the receptacle bodies has, on the inside, an edge seal (88, 118, 120) which interacts with a peripheral surface of the other receptacle body.

8. The applicator device as recited in any of the claims 1 to 7, **characterized in that** the cylindrical pin (18) has at least one annular groove which interacts with the edge seal (38) and defmes a deactivation position and/or an activation position of the receptacle device.

## Revendications

1. Dispositif d'application d'une substance fluide, comprenant un support (12), qui est pourvu d'un mécanisme d'application (14), et qui présente sur la partie désamorcée du mécanisme d'application (14) un tenon cylindrique (18), pénétré par un canal transversal (26), sur lequel s'embranche un canal axial (28), lui-même conduisant vers le mécanisme d'application (14), alors qu'un dispositif d'approvisionnement (30, 50, 60, 70, 80, 90, 100, 110) est monté de manière coulissante sur le tenon cylindrique (18), qui présente une lèvre d'étanchéité (38) annulaire sur l'intérieur, fonctionnant de manière étanche en accord avec le tenon cylindrique (18), alors que se produit une activation du dispositif d'application par la pression du dispositif d'approvisionnement (30, 50, 60, 70, 80, 100) sur le tenon cylindrique (18) en direction du mécanisme d'application (14), permettant une relation d'écoulement entre le dispositif d'approvisionnement (30, 50 , 60 , 70 , 80, 100, 110) et le canal transversal (26), **caractérisé en ce que** le dispositif d'approvisionnement présente au moins une section de réception flexible en partie élastique (34, 52, 62, 72, 82, 112) de telle manière que lorsqu'une relation d'écoulement existe entre le canal transversal (26) et la section de réception (34, 52, 62, 72, 82, 112) lors de l'activation du dispositif d'application, la substance fluide passe par le mécanisme d'application (14) grâce à une compression manuelle de la partie élastique flexible de la section de réception (34, 52, 62, 72, 82, 112).

2. Dispositif d'application selon revendication 1, **caractérisé en ce que** la portion élastique flexible de la section de réception (52, 72, 82) est en forme de vessie.

3. Dispositif d'application selon revendication 1, **caractérisé en ce que** la portion élastique flexible de la section de réception est en forme de petit tuyau.

4. Dispositif d'application selon revendication 1, **caractérisée en ce que** la portion élastique flexible de la section de réception est en forme de tube.

5. Dispositif d'application selon l'une des revendications 1 à 4, **caractérisée en ce que** le conduit (32) a une partie adaptée évasée (54), qui se situe à la hauteur du canal transversal (26) du tenon (18) lors de l'activation du dispositif d'application.

6. Dispositif d'application selon l'une des revendications 1 à 5, **caractérisé en ce que** le dispositif d'approvisionnement (80, 10, 110) comprend au moins deux réceptacles pour chaque composant d'un système à composants multiples.

7. Dispositif d'application selon revendication 6, **caractérisé en ce que** les 2 réceptacles minimums, destinés à la production d'une relation d'écoulement sont télescopables, avec au moins l'un des réceptacles muni à l'intérieur d'une lèvre d'étanchéité (88, 118, 120), combinée avec une superficie périphérique de l'autre réceptacle.

8. Dispositif d'application selon l'une des revendications 1 à 7, **caractérisé en ce que** le tenon cylindrique (18) présente au moins une rainure à anneaux qui fonctionne avec la lèvre d'étanchéité (38) et détermine une désactivation et/ou une activation du dispositif de réception.
